# EUROPEAN PATENT SPECIFICATION

(11) **EP 3 120 149 B1**
(45) Date of publication and mention of the grant of the patent: **11.09.2019**
(21) Application number: 15713566.6
(22) Date of filing: 20.03.2015
(51) Int. Cl.: G01N 33/68, A61K 31/00

(54) **ATRIAL FIBRILLATION THERAPY**
THERAPIE FÜR VORHOFFLIMMERN
TRAITEMENT DE LA FIBRILLATION AURICULAIRE

(30) Priority: 20.03.2014 GB 201404998
(43) Date of publication of application: 25.01.2017
(73) Proprietor: The University Of Birmingham, Birmingham B15 2TT (GB)
(72) Inventor: KIRCHHOF, Paulus, Birmingham West Midlands B15 2TT (GB); FABRITZ, Larissa, Birmingham West Midlands B15 2TT (GB); SYEDA, Fahima, Birmingham West Midlands B15 2TT (GB)
(74) Representative: Chapman, Paul Gilmour
(86) International application number: PCT/GB2015/050828
(87) International publication number: WO 2015/140571

(56) References cited:
- EP-A1- 2 213 749
- WO-A1-2004/002449
- ALEXANDER BURASHNIKOV ET AL: "Atrial-selective sodium channel block for the treatment of atrial fibrillation", EXPERT OPINION ON EMERGING DRUGS, INFORMA HEALTHCARE, UK, vol. 14, no. 2, 1 January 2009 (2009-01-01), pages 233-249, XP009140792, ISSN: 1472-8214
- XB QIU ET AL: "PITX2C loss-of-function mutations responsible for idiopathic atrial fibrillation", CLINICS, vol. 69, no. 01, 1 January 2014 (2014-01-01), pages 15-22, XP055188265, ISSN: 1807-5932, DOI: 10.6061/clinics/2014(01)03
- A. CHINCHILLA ET AL: "PITX2 Insufficiency Leads to Atrial Electrical and Structural Remodeling Linked to Arrhythmogenesis", CIRCULATION: CARDIOVASCULAR GENETICS, vol. 4, no. 3, 21 April 2011 (2011-04-21), pages 269-279, XP055188270, ISSN: 1942-325X, DOI: 10.1161/CIRCGENETICS.110.958116
- PETER C. KAHR ET AL: "Systematic Analysis of Gene Expression Differences between Left and Right Atria in Different Mouse Strains and in Human Atrial Tissue", PLOS ONE, vol. 6, no. 10, 19 October 2011 (2011-10-19), page e26389, XP055188268, DOI: 10.1371/journal.pone.0026389

## Description

### Field of the Invention

The present invention relates to methods according to the claims selectively determining suitable treatments for subjects suffering from atrial fibrillation, as well as the use a sodium channel blocker, such as flecainide or propafenone to treat a specific cohort of patients with atrial fibrillation.

### Background to the Invention

Atrial fibrillation (AF) is a common cause of stroke, death, heart failure, and hospitalizations in Europe and in the world. Current management strategies are not sufficient to prevent these complications of the arrhythmia. Rhythm control therapy, i.e. restoration of normal sinus rhythm, is often used in symptomatic patients, and may in the future gain a prognostic role in the management of AF. While catheter ablation emerges as a relatively effective but very invasive method to maintain sinus rhythm, antiarrhythmic drugs are the mainstay therapy of rhythm control in AF patients. Currently, there is no guidance on selecting an effective antiarrhythmic drug. Rather, the selection follows safety considerations at present (See Camm et al EurHJ 2010 and Camm et al EurHJ 2012 for ESC guidelines on atrial fibrillation). There is a clear need to identify patients who will respond to antiarrhythmic drug therapy in light of the relatively high recurrence rates of AF on such drugs, and also in view of the rare but potentially dangerous side effects of therapy.

Genome-wide association studies have consistently identified single nucleotide polymorphisms on chromosome 4q25, close to the pitx2 gene, to be associated with atrial fibrillation. Others have published that the response to antiarrhythmic drugs is different in carriers of the rare allele of the most common polymorphism on chromosome 4q25 (Parvez et al JACC 2012). Kirchhof et al (2011) have shown that mice with reduced pitx2c expression levels (pitx2c+/- mice) are more likely to develop atrial fibrillation. This has been confirmed by others (Wang PNAS 2010). Burashnikov et al (2009) discloses atrial-selective sodium channel block for the treatment of atrial fibrillation.

However, there has hitherto not been any suggestion of how the expression levels of Pitx2c could be associated with a response to antiarrhythmic drug therapy.

### Summary of the Invention

The present invention is based upon studies carried out by the present inventors into the effect reduced pitx2c expression has on response to antiarrhythmic drug therapy.

In a first aspect the present invention provides a method according to claim 1 of facilitating the determination of treatment of a subject displaying atrial fibrillation, the method comprising determining a level of pitx2c expression in a sample from the subject and selecting a treatment based upon the level of pitx2c expression, wherein treatment with a sodium channel blocker is selected if the level of pitx2c is determined to be below a reference level of a predetermined threshold.

In this regard, there is also provided a sodium channel blocker for use in a method according to claim 7 of treating atrial fibrillation wherein the subject to be treated displays a level of pitx2c which is below a reference level or a predetermined threshold.

A method of administering an antiarrhythmic drug to a subject in need of therapy may comprise the steps of:
identifying a subject with an atrial fibrillation in need of therapy;
determining a level of pitx2c expression in the subject and identifying if the level is elevated, approximately the same or reduced in comparison to a reference level; and
administering a sodium channel blocking antiarrhythmic drug to a subject with a reduced pitx2c expression level as compared to the reference level, (or administering another class of antiarrhythmic drug (e.g. a potassium channel blocker such as sotalol) to a subject with an elevated or approximately the same pitx2c expression level compared to the reference level).

A method of administering an antiarrhythmic drug to a subject displaying atrial fibrillation may comprise the steps of:
determining a level of pitx2c expression in a subject displaying atrial fibrillation; and
administering a sodium channel blocking antiarrhythmic drug to a subject with a reduced pitx2c expression level, or a subject with a pitxc2 expression level which is below a predetermined threshold (or administering another class of antiarrhythmic drug (e.g. a potassium channel blocker such as sotalol) to a subject not having a reduced pitx2c expression level, or a subject having a pitx2c expression level which is approximately equal to or above a predetermined threshold).

A method of administering an antiarrhythmic drug for the treatment of atrial fibrillation in a subject, may comprise:
administering a sodium channel blocker, as the antiarrhythmic drug for the treatment of atrial fibrillation, to a subject displaying a reduced level of pitx2c expression as compared to a reference level or a predetermined threshold.

There is further described a method comprising:
determining a level of pitx2c expression in sample from a subject displaying atrial fibrillation; and
selecting an antiarrhythmic drug for use in a method of treating atrial fibrillation; wherein the antiarrhythmic drug is a sodium channel blocker when the pitx2c expression level is reduced or below a predetermined threshold level; or a potassium channel blocker wherein the pitx2c expression level is elevated, approximately equal to or above a predetermined threshold level.

Conveniently the biological sample may be any appropriate fluid or tissue sample obtained from the subject. For example, the biological sample may comprises at least one of the group consisting of: urine, saliva, blood, sputum, semen, faeces, a nasal swab, tears, a vaginal swab, a rectal swab, a cervical smear, a tissue biopsy, and a urethral swab. Suitably, the biological sample is one that can be readily obtained from the individual, such as urine, saliva, blood and sputum, which the individual may be able to collect from him/herself, without the need for assistance. In some embodiments the biological sample is blood. Similarly, a sample from a subject not displaying atrial fibrillation can be obtained by any method known in the art. In order to provide a reference level of pitx2c expression, or a "normal" level of pitx2c expression determined or provided for use in determining whether or not the level of pitx2c expression in the subject being tested is higher or lower than "normal" or from a subject not displaying atrial fibrillation.

It will be appreciated that pitx2c expression may be measured directly, or indirectly via a pitx2c-dependent gene or gene-product.

A "reference level" for pitx2c expression as used herein, is so determined or provided in order that the subject with a pitx2c expression level lower or higher than the predetermined value can be determined and wherein the subject is likely to experience a more or less desirable clinical outcome to a particular treatment than patients with pitx2c expression levels higher or lower or approximately the same as the predetermined value, or vice-versa. In accordance with the present invention pitx2c expression levels lower than normal, are associated with a favourable clinical outcome when the subject is treated with a sodium channel blocker as defined herein.

"Lower" or "higher" (or reduced or elevated) expression refers to increased or decreased expression, as compared to the expression level of pitx2c in a control sample.

The control sample is a taken from a non-diseased subject, or is a published literature value for expected normal pitx2c expression. In one aspect, the differential expression, that is higher or lower may be about 0.5 times, 1 times, 1.5 times, or alternatively, about 2.0 times, or alternatively, about 2.0 times, or alternatively, about 3.0 times, or alternatively, about 5 times, or alternatively, about 10 times, or alternatively about 50 times, or yet further alternatively more than about 100 times higher or lower than the expression level in the control sample or published value. Alternatively, pitx2c may be referred to as "over expressed" or "under expressed". Alternatively, pitx2c may also be referred to as "up regulated" or "down regulated".

The expression level of pitx2c is used as a basis for selecting a treatment as described herein. The expression level may be measured before and/or during treatment and the values obtained may be used by a clinician in assessing any of the following: (a) probable or likely suitability of an individual to initially receive treatment; (b) probable or likely unsuitability of an individual to initially receive treatment; (c) responsiveness to treatment; (d) probable or likely suitability of an individual to continue to receive treatment; (e) probable or likely unsuitability of an individual to continue to receive treatment; (f) adjusting dosage; (g) predicting likelihood of clinical benefits; or (h) toxicity. As would be well understood by one in the art, measurement of the pitx2c expression level in a clinical setting is a clear indication that this parameter was used as a basis for initiating, continuing, adjusting and/or ceasing administration of the treatments described herein.

The sodium channel blocker is typically a type 1 antiarrhythmic drug, more preferably a type 1c antiarrhythmic drug. The sodium channel blocker may be flecainide, propafenone, ranolazine, vernakalant or quinidine, or derivatives thereof. Preferred sodium channel blocking drugs are flecainide and propafenone.

The pitx2c expression level to be determined may be a protein level or mRNA level. Thus, the present invention is distinguished over methods concerned simply with the determination of genotype. Assessment of protein expression levels is routine in the art. For example, one method of measuring protein levels is via Western blotting or immunohistochemistry using antibodies to pitx2c. The inventors have observed that there is a direct correlation between the expression level of pitx2c and the likelihood that an individual will be responsive to treatment with a sodium channel blocking drug. Consequently, the sensitivity of the protein assay is particularly important. Therefore, radioimmunoassay (RIA), enzyme-linked immunosorbent assay (ELISA), flow cytometry, or any other more sensitive quantitative method of measuring pitx2c is preferably used instead of less quantitative methods. In a preferred embodiment, the expression level is determined by an immunological method, preferably using a solid-phase antibody, an ELISA or ELISPOT assay.

In other embodiments, an mRNA expression level may be determined by nucleic acid hybridization (Northern Blotting) or nucleic acid amplification. In preferred embodiments, the nucleic acid hybridization is performed using a solid-phase nucleic acid molecule array. In other preferred embodiments, the nucleic acid amplification method is reverse transcriptase PCR (RT-PCR). Two common methods for the detection of products in RT-PCR are: (1) non-specific fluorescent dyes that intercalate with any double-stranded DNA, and (2) sequence-specific DNA probes consisting of oligonucleotides that are labelled with a fluorescent reporter which permits detection only after hybridization of the probe with its complementary sequence to quantify messenger RNA (mRNA). Further details with regards to RT-PCR will be known to those skilled in the art and can be found in common laboratory manuals (e.g. Sambrook and Russell, Molecular Cloning: A laboratory Manual, CSHL Press, 2001).

The level of pitx2c expression may be determined directly (i.e. by determining the pitx2c protein or mRNA level) or indirectly, by determining the level of a biomarker which is indicative of the level of pitx2c expression.

Thus, in some embodiments, the methods of the invention comprise determining a level of a biomarker in a sample obtained from a subject, wherein the level of biomarker is indicative of the level of pitx2c expression. In some embodiments, the method may comprise selecting a treatment based on the level of the biomarker. In some embodiments, the method may comprise selecting treatment with a sodium channel blocking drug if the biomarker level is below a predetermined threshold. In some further embodiments, the method may comprise determining or predicting the pitx2c expression level based on the level of the biomarker.

In some embodiments, the method may comprise:
determining the level of the biomarker, and
optionally, determining the pitx2c level based on the level of the biomarker.

A sodium blocking channel antiarrhythmic drug can be administered to a subject displaying a level of biomarker which is below a predetermined threshold, or a subject determined to have a reduced level of pitx2c expression as compared to a reference level or a predetermined threshold.

In some embodiments, the biomarker is ccl21.

In accordance with the invention, the use of an assay system in the method of the invention is provided. The assay system includes a measurement device that measures pitx2c expression (either directly by measuring pitx2c mRNA or protein, or indirectly by measuring a suitable biomarker) in order to provide data in relation to the level of pitx2c expression. The system also includes a data transformation device that acquires the pitx2c expression data from the measurement device and performs data transformation to calculate whether or not the level determined is higher or lower than a reference or normal value for pitx2c expression.

In other embodiments, the assay system also includes a user interface output device to output data to a user. In other preferred embodiments, the assay system also includes a database of treatment information, wherein the device identifies treatment information in the database for the level of pitx2c determined and outputs the treatment information to the user interface output device.

An apparatus for determining the treatment of a subject displaying AF may comprise a measurement device for measuring pitx2c expression, or an assay system comprising such a measurement device. The apparatus may be suitable for use at the point of care, for example in a doctor's surgery or at a patient's bedside. Conveniently, the apparatus may be portable.

In a further aspect there is provided a kit for use in the present methods. The kit can comprise at least one antibody, probe and/or primer which is/are capable of specifically binding to pitx2c mRNA or protein, or a biomarker indicative of pitx2c expression, and may be labeled for example with a fluorescent or luminescent label and instructions for use. Preferred kits for amplifying at least a portion of pitx2c, or a biomarker thereof, comprise two primers.

Oligonucleotides, whether used as probes or primers, contained in a kit can be detectably labeled. Labels can be detected either directly, for example for fluorescent labels, or indirectly. Indirect detection can include any detection method known to one of skill in the art, including biotin-avidin interactions, antibody binding and the like. Fluorescently labeled oligonucleotides also can contain a quenching molecule. Oligonucleotides or antibodies can be bound to a surface. In one embodiment, the preferred surface is silica or glass. In another embodiment, the surface is a metal electrode.

### Detailed description

Embodiments of the invention will now be described by way of example and with reference to the accompanying Figures, in which:
Figure 1 is an illustrative example of electrophysiological signals recorded from a Pitx2c^{+/-} mouse heart, namely an atrial monophasic action potential (top tracing, MAP), an intracardiac electrogram recorded from the atrial (middle tracing) and an electrogram recorded from the right ventricle (bottom tracing). Programmed stimulation induced atrial fibrillation at baseline (top tracing), but fails to do so during perfusion with the sodium channel blocker flecainide (bottom tracing);
Figure 2 is a graph showing the number of Pitx2c^{+/-} and wild-type (WT) hearts with inducible atrial arrhythmias elicited with a single atrial premature stimulus, with or without flecainide perfusion. Wild type hearts do not show a difference in inducible arrhythmias. In pitx2c+/- hearts, flecainide completely suppresses atrial arrhythmias;
Figure 3 is a plot showing the mean action potential durations (APD) measured by monophasic action potential catheters at different paced cycle lengths in Pitx2c^{+/-} and WT hearts;
Figure 4 is a plot showing the mean action potential durations (APD) in Pitx2c^{+/-} and WT hearts before and after perfusion with flecainide at three different paced cycle lengths (80 - 100 - 120 ms);
Figure 5 is a graph showing the change in inter-atrial activation time in Pitx2c^{+/-} and WT hearts after flecainide perfusion. Flecainide has a pronounced effect on activation times in pitx2c+/- mice, but a much lesser effect in wild-type mice; and
Figure 6 is a plot showing the change in effective refractory period (ERP) in Pitx2c^{+/-} and WT hearts after flecainide perfusion. Flecainide increases effective refractory periods in Pitx2c+/- mice much more than in wild-type mice.

### Example 1: Comparison of the effect of antiarrhythmic drugs on hearts of wild-type mice and mice with reduced pitx2c expression

### Example 1.1: Flecainide

### Methods

The inventors studied the effects of the sodium channel blocker flecainide in the isolated, beating heart of wild type mice and mice with a heterozygous deletion of the Pitx2c gene. This mouse model (provided by Nigel Brown, St George's London) is an established model for reduced Pitx2 mRNA expression that is susceptible to AF. In PITX2c^{+/-} mice, pitx2c mRNA expression was found to be reduced to about 60% of the WT level in the left atrium (Kirchhof et al., Circ Cardiovasc Genet. 2011;4:123-133).

### Electrophysiological study in the isolated heart

PITX2c^{+/-} and WT hearts (3-4 months old), were rapidly excised and Langendorff-perfused to record left atrial (LA) monophasic action potential duration. In this preparation, the beating heart is perfused with a warm, oxygenated modified Krebs-Henseleit solution containing (in mmol/l): NaCl 118; NaHCO₃ 24.88; KH₂PO 1.18; Glucose 5.55; Na-Pyruvate 5; MgSO₄ 0.83; CaCl₂ 1.8; KCl 3.52 (95% O₂-5% CO₂, pH 7.4) at constant perfusion pressure (100 ± 5 mmHg) and coronary flow (4 ± 0.5ml/min).

Thereby, detailed electrophysiological measurements can be taken for 1-2 hours in the beating, functioning, intact heart (perfusate temperature 37°C, perfusion flow rate 3-5 mL/min) (Fabritz et al., Basic Res Cardiol 2003;98:25-32). An octapolar murine electrophysiology catheter (0.5 mm electrode spacing, CIB'ER MOUSE, NuMED, Hopkinton, N.Y., USA) was inserted into the right atrium (RA) and right ventricle for pacing. Atrial and ventricular electrograms and monophasic action potential (MAP) from the left atrium were simultaneously recorded. After instrumentation, the hearts were subjected to a stabilization period of 10 minutes, followed by observation of spontaneous sinus rhythm for 5 minutes to provoke repolarization-related arrhythmias. Then the right atrium was paced at constant physiological heart rates (80-120 ms paced cycle length) for 1.5 minutes of pacing per heart rate to record steady-state action potential duration, and premature right atrial stimulation was performed using trains of 8 beats following by a premature stimulus in 2 ms steps to determine the effective refractory period (ERP) and inducibility of arrhythmias. Arrhythmias > 1 second were counted. The pacing protocol was repeated after adding flecainide acetate (1 µm) to the perfusate.

Experiments were accepted for analysis of action potential duration and activation time if they met published quality criteria and if MAPs were stable. Signals > mV were accepted for analysis of action potentials and activation times, and signals > 0.5 mV amplitude were accepted for evaluation of arrhythmias. Recordings were manually screened for arrhythmias. Atrial fibrillation was defined as >5 closely couple polymorphic consecutive atrial ectopic beats with a cycle length shorter than sinus-rhythm cycle length.

### Transmembrane potential recordings in isolated, superfused left atria

Transmembrane action potentials were recorded from isolated superfused left atria using borosilicate glass microelectrodes (tip resistance 15-30 MΩ), filled with 3 M KCl. Voltage signals were amplified (Axoclamp 2B; Molecular Devices, USA), digitised and displayed using spike2 software (Cambridge Electronic Design, UK) at 20kHz sampling frequency. LA were paced at different cycle lengths at 2 ms pulse width at 2x diastolic threshold through bipolar platinum electrodes. APD was analysed off line (spike 2, CED, UK). After 15 min pacing for equilibration, preparations were paced successively at increasing frequencies from 0.5 to 10 Hz, with 200 beats at each frequency to ensure steady state prior to recording.

### Modelling of the electrophysiological consequences of increased resting membrane potential.

The human atrial model of Courtemanche-Ramirez-Nattel for atrial cells was used to assess the effects of 0.5-1 µM flecainide. To reproduce the depolarised resting membrane potential and shorter action potential duration observed in the Pitx2c-deficient murine model, the maximal I_{K1} conductance was reduced by 25% and I_{Kr} conductance was doubled. The effect of flecainide on sodium channels was simulated by reducing maximal I_{Na}.conductance to 50% and 40%. Simulations were run in atrial cell strands of 100 excitable elements ("cells"; diameter 100 µm). To reach steady state, the 5 leftmost cells of the strand were paced (S1) for 2 minutes at 1000 and 500 ms paced cycle length. A premature stimulation (S2) was applied to determine the ERP and CV was measured from cells 25-75 of the model. Resting membrane potential was measured as the minimum potential of the cycle preceding that arising from the S2 stimulus. Values for all other parameters were measured from the 50^{th} cell. Post-repolarisation refractoriness is reported as a function of the diastolic interval in the 50^{th} cell.

### Statistical analysis

Categorical data were compared using Fishers exact test. Numerical data were compared by two sided paired t tests (e.g. measurements before and after perfusion of flecainide or sotalol) and Wilcoxon signed-rank tests. For multiple measurements data were analysed by repeated measures analysis of variance followed by a multiple comparison procedure (Bonferroni t-test) if the overall test was significant. Data were considered significantly different at two-sided p values <0.05. When data are displayed as boxplots, boxes and box limits indicate the data range mean, and standard error. Whiskers indicate the minimum and maximum of the respective data. Individual measurements are shown in the boxplots as points.

### Results

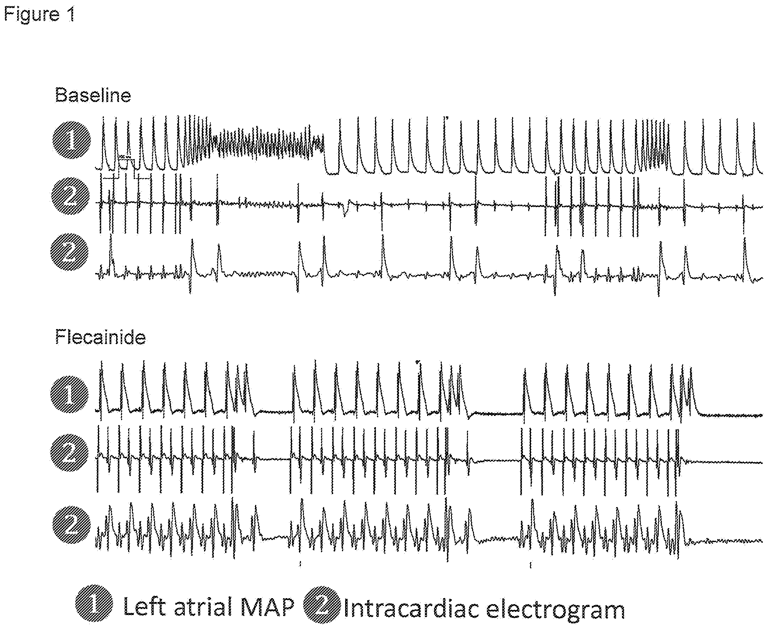

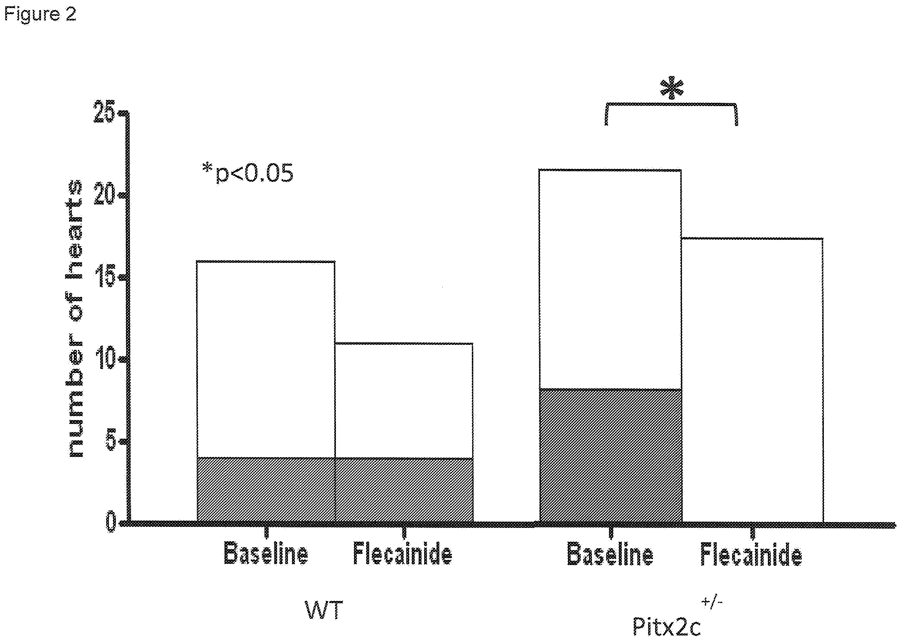

Figures 1 and 2 provide an illustrative example of a short episode of atrial fibrillation induced in a pitx2c+/- heart and the suppression of atrial fibrillation in the same heart by perfusion with flecainide. As shown in Figure 2, flecainide suppressed atrial fibrillation in all Pitx2c+/- hearts that showed atrial fibrillation at baseline (8/21). Flecainide did not suppress atrial fibrillation in mice with normal Pitx2c expression (wild type hearts, 4/16 hearts with arrhythmias at baseline, 4/11 with flecainide). This indicates that flecainide suppresses inducible atrial arrhythmias in Pitx2c^{+/-} mice.

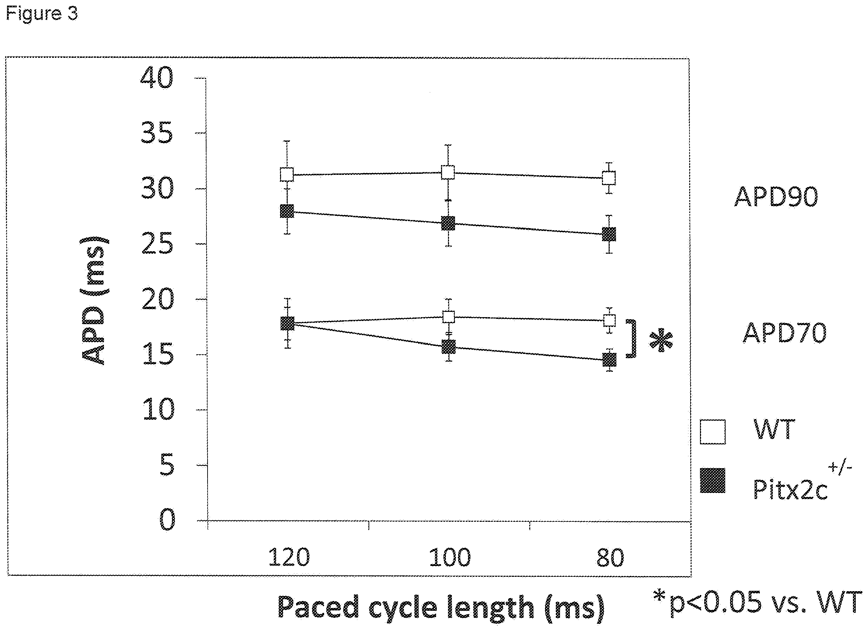

Pitx2c+/- mice have shorter action potential durations at high paced cycle lengths than their wild-type littermates (Figure 3).

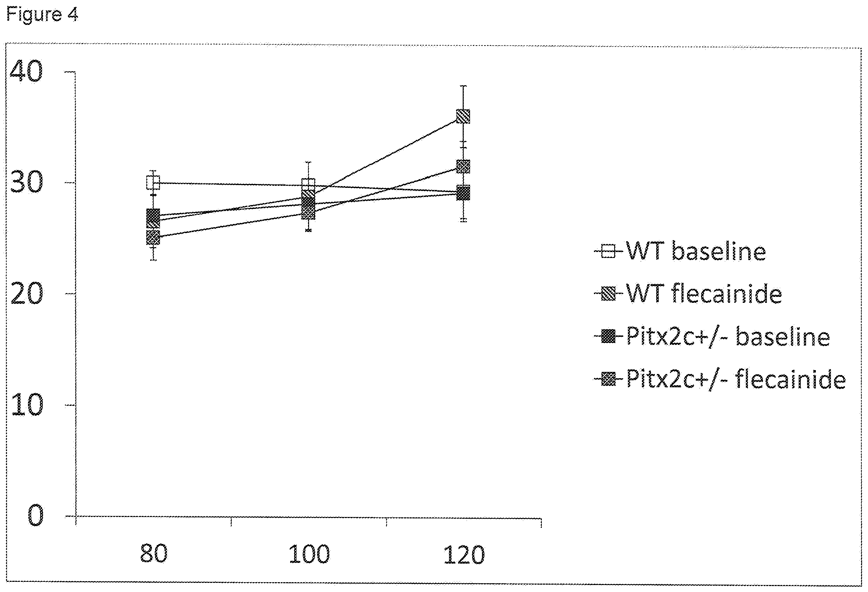

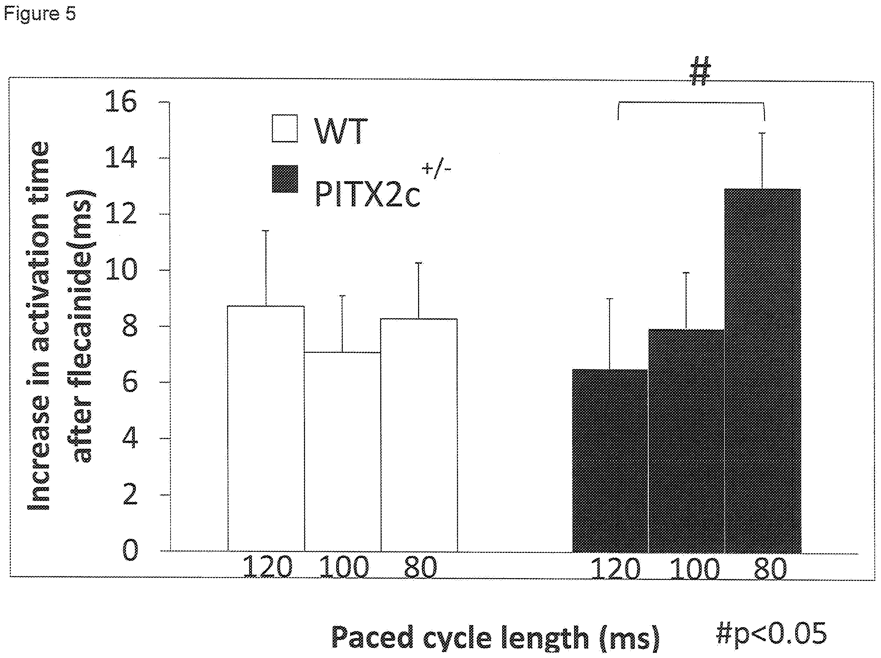

No difference was observed in the effect of flecainide on action potential duration (APD) between wild-type and Pitx2c+/- mice (Figure 4). This indicates that Flecainide does not alter atrial APD in either WT or PITX2c^{+/-} hearts. However, flecainide was found to have a marked effect on atrial activation times in Pitx2c+/- mice, while there is only a negligible effect in wild-type mice (Figure 5).

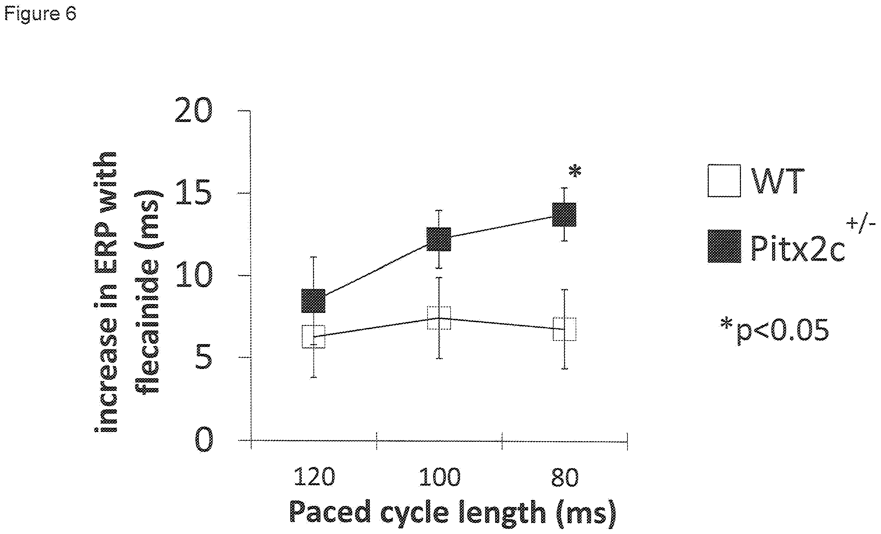

As shown in Figure 6, flecainide induces more post-repolarization refractoriness (i.e. prolongation of the refractory period beyond the end of the action potential) in Pitx2c+/- mice (e.g. 12 ms at 80 ms paced cycle length) compared to WT mice (e.g. 7.5 ms at 80 ms paced cycle length). Post-repolarization refractoriness is a powerful mediator of antiarrhythmic efficacy in large animals (Kirchhof Fabritz et al., Circulation 1998; Kirchhof et al., JPET 2003; Kirchhof P. et al., Heart Rhythm. (2012); Milberg P. et al., Europace. (2013); Milberg P. et al., Journal of Cardiovascular Electrophysiology. 18:658-664 (2007); Kirchhof P, et al., J Pharmacol Exp Ther. 305:257-263 (2003)) and in patients with atrial fibrillation (Kirchhof P, et al. Basic Res Cardiol. 100:112-121 (2005)).

There are only small differences in action potential duration and refractoriness at baseline between pitx2c+/- and WT atria, but these differences are markedly more pronounced during perfusion with flecainide (lower panels): The refractory period is dramatically prolonged, causing marked post-repolarization refractoriness.

The analysis described above was initially done blinded. After unblinding, the differential effects discussed above were identified.

Taken together, these findings demonstrate that the sodium channel blocker flecainide has a profoundly different effect in atria with reduced pitx2c expression compared to atria with normal pitx2c levels. It was not only found that flecainide is much more effective in reducing atrial fibrillation in mice with reduced pitx2c expression, but also identified were several relevant differences in the electrophysiological effects of flecainide in atria with reduced pitx2c expression, e.g. a higher post repolarization refractoriness (Figure 6).

Pitx2c^{+/-} left atria had significantly (p<0.05) more depolarised resting membrane potentials at all cycle lengths tested as measured by sharp electrodes inserted into superfused left atrial preparation, e.g. -68 ± 0.7 mV (n = 31 cells) when compared with wildtype (-70 ± 0.7mV, n = 30 cells) at 100ms paced CL. Flecainide did not alter resting membrane potential in either genotype. This can potentially explain the altered response to sodium channel blockers, as the binding of sodium channel blockers will be enhanced in cells that are more depolarized during electrical diastole.

When a more positive resting membrane potential was mimicked in the Courtemanche model of human atrial cardiomyocytes, flecainide did not alter the resting membrane potential, but sodium channels remain in the closed state for longer, and this was more pronounced in the presence of flecainide and during rapid pacing. Flecainide also had an enhanced effect on PRR prolongation when the resting membrane potential was increased compared to the reference model. Thus, the modelling of human atrial tissue replicated our findings in mice.

### Example 1.2: Sotalol

Further experiments suggested that the antiarrhythmic effect of the potassium channel blocker d,l sotalol is lost in Pitx2c+/- hearts: In three pairs of mice studied using the same experimental setup described above, sotalol (10 µM) prolonged atrial APD in WT hearts, but did not alter atrial APD in Pitx2c+/- hearts. These initial observations provide an explanation why potassium channel blockers such as sotalol may not be effective antiarrhythmic drugs in patients with reduced atrial pitx2c mRNA expression, and lend further support to the determination of pitx2c mRNA levels to aid the selection of antiarrhythmic drugs in patients with atrial fibrillation.

### Conclusions

The present inventors have found that the effects of the antiarrhythmic drug flecainide are profoundly altered in the left atria of mice with reduced pitx2c expression compared to the wild-type mice. The effective refractory period was prolonged to a much higher value in PITX2c^{+/-} hearts, and most significantly flecainide was more effective in preventing AF in PITX2c^{+/-} mouse hearts compared to the wild type hearts. These observations indicate that patients with reduced pitx2c mRNA expression are suitable for therapy with a sodium channel blocking antiarrhythmic drug, such as flecainide, while others may possibly benefit from treatment with another agent, e.g. a potassium channel blocker such as sotalol.

It was found that PITX2c^{+/-} left atria have reduced expression of some potassium channel genes, which may contribute to their higher resting membrane potentials and shorter action potential duration. This effect can explain the altered response to sodium channel blockers, as demonstrated in a computer model of human action potentials.

The invention provides for the first time a test that allows one to determine the effectiveness of a class of antiarrhythmic drug in the prevention of atrial fibrillation at the time point of initiation of such therapy. The method of the invention will enable the identification of subjects who will benefit from treatment with a sodium channel blocker, and thereby aids clinicians in selecting the most appropriate medication.

### Example 2: PITX2 expression levels in human atria

RNA was prepared using Qiagen RNeasy fibrous mini-kit and Quiagen QiaShredder Columns. cDNA was generated and RT-PCR reactions were performed using gene specific primers and SYBR Green (Life Technologies) on an ABI 7500 Fast machine.

We assessed the expression levels of PITX2 mRNA in human left and right atria, and could confirm that PITX2 mRNA is mainly expressed in the left atrium. A clear variation (up to 100-fold) in the expression levels of PITX2 mRNA in human left atria was observed: In some left atria, PITX2 expression was undetectable. More often, PITX2 expression levels are between 1% and 4% of actin levels. In some patients, left atrial PITX2 expression reaches up to 10% of actin expression. These data clearly illustrate that PITX2 expression in the left atrium varies in patients, rendering a selection of therapy based on PITX2 levels useful for the treatment of patients with atrial fibrillation.

### Example 3: Use of pitx2c level to select a treatment for atrial fibrillation

A biological sample (e.g. blood) obtained from a subject displaying atrial fibrillation is analysed and the level of pitx2c mRNA is measured. The pitx2c mRNA level in the sample is compared to a pre-determined reference level. A suitable anti-arrhythmic drug is selected and administered to the subject depending on whether the measured pitx2c mRNA level in the sample is higher, lower or approximately the same as the pre-determined reference level.

If it is determined that the pitx2c mRNA level is reduced (i.e. the measured pix2c mRNA level is lower than the reference level) such that the subject is identified as having atrial fibrillation which is associated with reduced pitx2c expression, a sodium channel blocking drug (such as flecainide) can be administered. If it is determined that the pitx2c mRNA level is not reduced relative to the reference level, a potassium channel blocker (e.g. sotalol) can be administered to the subject.

## Claims

1. An in vitro method of facilitating the determination of treatment of a subject displaying atrial fibrillation, the method comprising determining a level of pitx2c expression in a sample obtained from the subject and selecting a treatment based upon the level of pitx2c expression, wherein treatment with a sodium channel blocker is selected if the level of pitx2c expression is determined to be reduced or below a predetermined threshold, which is the expression level of pitx2c in a control sample from a non-diseased subject or a published literature value for expected normal pitx2c expression.

2. The method of claim 1, wherein the sample comprises at least one of urine, saliva, blood, sputum, semen, faeces, a nasal swab, tears, a vaginal swab, a rectal swab, a cervical smear, a tissue biopsy, and a urethral swab.

3. The method of claim 2, wherein the sample is blood.

4. The method of any one of the preceding claims, wherein the level of pitx2c expression is determined directly by determining the pitx2c protein level or mRNA level.

5. The method of any one of claims 1 to 3, wherein the level of pitx2c expression is determined indirectly by determining the level of a biomarker which is indicative of the level of pitx2c expression.

6. The method of claim 5, wherein the biomarker is ccl21.

7. A sodium channel blocker for use in a method of treating atrial fibrillation wherein the subject to be treated displays a reduced level of pitx2c expression, or a level of pitx2c which is below a predetermined threshold, which is the expression level of pitx2c in a control sample from a non-diseased subject or a published literature value for expected normal pitx2c expression.

8. The method of any one of claims 1 to 6, or the sodium channel blocker for use in a method according to claim 7, wherein the sodium channel blocker is a type 1 antiarrhythmic drug.

9. The method of any one of claims 1 to 6, or the sodium channel blocker for use in a method according to claim 7, wherein the sodium channel blocker is flecainide or propafenone.

10. In vitro use of an assay system in the method of any one of claims 1 to 6, wherein the assay system comprises:
a measurement device that measures pitx2c expression; and
a data transformation device that acquires the pitx2c expression data from the measurement device and performs data transformation to calculate whether or not the level determined is higher or lower than a reference or normal value for pitx2c expression.

11. The use of the assay system of claim 10, wherein the assay system further comprises a user interface output device to output data to a user.

12. The use of the assay system of claim 10 or claim 11, wherein the assay system further comprises a database of treatment information, wherein the device identifies treatment information in the database for the level of pitx2c determined and outputs the treatment information to the user interface output device.

## Patentansprüche

1. *In vitro*-Verfahren zum Erleichtern der Bestimmung einer Behandlung eines Subjekts, das Vorhofflimmern zeigt, wobei das Verfahren Bestimmen eines Werts der Pitx2c-Expression in einer Probe umfasst, die vom Patienten erhalten wird, und Auswählen einer Behandlung, die auf dem Wert der Pitx2c-Expression beruht, wobei eine Behandlung mit einem Natriumkanalblocker ausgewählt wird, wenn festgestellt wird, dass der Wert der Pitx2c-Expression unterhalb eines Referenzwerts oder eines vorbestimmten Schwellenwerts liegt, welcher der Wert der Expression von Pitx2c in einer Kontrollprobe von einem nicht erkrankten Patienten oder ein publizierter Literaturwert für erwartete normale Pitx2c-Expression ist.

2. Verfahren nach Anspruch 1, wobei die Probe mindestens eines von Urin, Speichel, Blut, Sputum, Samen, Kot, einem Nasentupfer, Tränen, einem Vaginaltupfer, einem Rektaltupfer, einem Zervix-Abstrich, einer Gewebebiopsie und einem Harnröhrenabstrich umfasst.

3. Verfahren nach Anspruch 2, wobei die Probe Blut ist.

4. Verfahren nach einem der vorhergehenden Ansprüche, wobei der Wert der Pitx2c-Expression direkt bestimmt wird, indem der Pitx2c-Proteinwert oder des mRNA-Wert bestimmt werden.

5. Verfahren nach einem der Ansprüche 1 bis 3, wobei der Wert der Pitx2c-Expression indirekt bestimmt wird, indem der Wert eines Biomarkers bestimmt wird, der für den Wert der Pitx2c-Expression kennzeichnend ist.

6. Verfahren nach Anspruch 5, wobei der Biomarker ccl21 ist.

7. Natriumkanalblocker zur Verwendung in einem Verfahren zum Behandeln von Vorhofflimmern, wobei das Subjekt, das behandelt werden soll, einen Wert von Pitx2c-Expression zeigt, der unterhalb eines Referenzwert oder eines vorbestimmten Schwellenwerts liegt, welcher der Wert der Expression von Pitx2c in einer Kontrollprobe von einem nicht erkrankten Patienten oder ein publizierter Literaturwert für erwartete normale Pitx2c-Expression ist.

8. Verfahren nach einem der Ansprüche 1 bis 6 oder der Natriumkanalblocker zur Verwendung in einem Verfahren nach Anspruch 7, wobei der Natriumkanalblocker ein antiarrhythmischer Arzneistoff vom Typ 1 ist.

9. Verfahren nach einem der Ansprüche 1 bis 6 oder der Natriumkanalblocker zur Verwendung in einem Verfahren nach Anspruch 7, wobei der Natriumkanalblocker Flecainid oder Propafenon ist.

10. *In vitro*-Verwendung eines Assay-Systems in dem Verfahren nach einem der Ansprüche 1 bis 6, wobei das Assay-System umfasst:
eine Messvorrichtung, die die Pitx2c-Expression misst; und
eine Vorrichtung zur Datentransformation, welche die Daten der Pitx2c-Expression von der Messvorrichtung erfasst und eine Datentransformation ausführt, um zu berechnen, ob der Wert, der bestimmte worden ist, höher oder niedriger als ein Referenz- oder Normalwert für Pitx2c-Expression ist oder nicht.

11. Verwendung des Assay-Systems nach Anspruch 10, wobei das Assay-System ferner eine Benutzeroberflächen-Ausgabevorrichtung umfasst, um Daten an einen Benutzer auszugeben.

12. Verwendung des Assay-Systems nach Anspruch 10 oder Anspruch 11, wobei das Assay-System ferner eine Datenbank mit Behandlungsinformation umfasst, wobei die Vorrichtung Behandlungsinformationen in der Datenbank für den bestimmten Wert von Pitx2c identifiziert und die Behandlungsinformation an die Benutzeroberflächen-Ausgabevorrichtung ausgibt.

## Revendications

1. Procédé *in vitro* pour faciliter la détermination de traitement d'un sujet présentant une fibrillation auriculaire, le procédé comprenant la détermination d'un taux d'expression de pitx2c dans un échantillon obtenu auprès du sujet et le choix d'un traitement reposant sur le taux d'expression de pitx2c, dans lequel un traitement avec un bloqueur des canaux sodiques est choisi si le taux d'expression de pitx2c est déterminé comme étant en dessous d'un taux de référence ou d'un seuil prédéterminé, qui est le taux d'expression de pitx2c dans un échantillon témoin d'un sujet non malade ou une valeur publiée dans la littérature pour une expression de pitx2c normale attendue.

2. Procédé selon la revendication 1, dans lequel l'échantillon comprend au moins l'un parmi l'urine, la salive, le sang, un crachat, le sperme, les selles, un prélèvement nasal, les larmes, un frottis vaginal, un prélèvement rectal, un frottis du col de l'utérus, une biopsie tissulaire et un prélèvement urétral.

3. Procédé selon la revendication 2, dans lequel l'échantillon est du sang.

4. Procédé selon l'une quelconque des revendications précédentes, dans lequel le taux d'expression de pitx2c est déterminé directement en déterminant le taux de protéine pitx2c ou le taux d'ARNm.

5. Procédé selon l'une quelconque des revendications 1 à 3, dans lequel le taux d'expression de pitx2c est déterminé indirectement en déterminant le taux d'un biomarqueur qui est indicatif du taux d'expression de pitx2c.

6. Procédé selon la revendication 5, dans lequel le biomarqueur est ccl21.

7. Bloqueur des canaux sodiques pour l'utilisation dans un procédé de traitement d'une fibrillation auriculaire, dans lequel le sujet devant être traité présente un taux d'expression de pitx2c qui est en dessous d'un taux de référence ou d'un seuil prédéterminé, qui est le taux d'expression de pitx2c dans un échantillon témoin d'un sujet non malade ou une valeur publiée dans la littérature pour une expression de pitx2c normale attendue.

8. Procédé selon l'une quelconque des revendications 1 à 6, ou bloqueur des canaux sodiques pour l'utilisation dans un procédé selon la revendication 7, dans lequel le bloqueur des canaux sodiques est une substance médicamenteuse antiarythmique de type 1.

9. Procédé selon l'une quelconque des revendications 1 à 6, ou bloqueur des canaux sodiques pour l'utilisation dans un procédé selon la revendication 7, dans lequel le bloqueur des canaux sodiques est la flécaïnide ou la propafénone.

10. Utilisation *in vitro* d'un système d'essai dans le procédé selon l'une quelconque des revendications 1 à 6, dans laquelle le système d'essai comprend:
un dispositif de mesure qui mesure l'expression de pitx2c; et
un dispositif de transformation des données qui acquiert les données d'expression de pitx2c à partir du dispositif de mesure et effectue une transformation des données pour calculer si le taux déterminé est supérieur ou inférieur ou non à une valeur de référence ou normale pour l'expression de pitx2c.

11. Utilisation du système d'essai selon la revendication 10, dans laquelle le système d'essai comprend en outre un dispositif en sortie avec une interface utilisateur pour fournir les données à un utilisateur.

12. Utilisation du système d'essai selon la revendication 10 ou la revendication 11, dans laquelle le système d'essai comprend en outre une base de données d'informations de traitement, dans laquelle le dispositif identifie les informations de traitement dans la base de données pour le taux de pitx2c déterminé et fournit les informations de traitement au dispositif en sortie avec une interface utilisateur.
